# EUROPEAN PATENT APPLICATION

(11) **EP 1 205 751 A2**
(43) Date of publication of application: **15.05.2002**
(21) Application number: 01125505.6
(22) Date of filing: 25.10.2001
(51) Int. Cl.: G01N 33/14, G01N 33/00

(54) **Device and procedure for checking the deterioration of the organoleptic characteristics of a drink**

(30) Priority: 08.11.2000 IT MI002415
(71) Applicant: De' Longhi S.p.A., 31100 Treviso (IT)
(72) Inventor: De' Longhi, Giuseppe, 31100 Treviso (IT)
(74) Representative: Rapisardi, Mariacristina

(57) **Abstract**

A device (1) for checking the deterioration of the organoleptic characteristics of a drink comprises means for detecting (2) the amount of drink present in a vessel (3), timing means (9), activated by detecting means (2), and indicating means (10) of the progressive deterioration of the organoleptic characteristics, activated by said timing means (9).

The procedure for checking the deterioration of the organoleptic characteristics of a drink consists of periodically detecting the amount of a drink present in a vessel and of then indicating, as a function both of the detected amount and of the time which has passed since the preparation of said drink, the progressive deterioration of the organoleptic properties of the same drink.

## Description

The present invention refers to a device and a procedure for checking the deterioration of the organoleptic characteristics of a drink.

In particular, hereafter reference will be made to drinks prepared through percolation, for example coffee-based drinks, although it is clear that analogous considerations are valid for any drinks, for example tea.

Currently a certain type of coffee drink, normally referred to as "filter coffee", is prepared in machines equipped with a vessel in which the drink itself accumulates.

After preparation, the coffee is usually left in the vessel and kept hot until it is consumed.

However, coffee, kept stored in the vessel and undergoing constant heating, undergoes a rapid degenerative process which causes a deterioration of its organoleptic qualities and, in practice, of the quality known as aroma making it, after a certain period of time, qualitatively inferior.

The technical task asked of the present invention is that of realising a device and a procedure for checking the deterioration of the organoleptic characteristics of a drink which indicates to the consumer if and to what degree the organoleptic characteristics of the drink have undergone a degenerative process, with the consequent deterioration of the quality of the drink.

In this technical task a purpose of the invention is that of realising a device which takes account, as well as of the length of time it takes for the organoleptic characteristics of the drink to deteriorate, also of the amount of the same drink which is present inside the vessel.

The technical task, as well as these and other purposes, according to the present invention are achieved by realising a device for checking the deterioration of the organoleptic characteristics of a drink, characterised in that it comprises means for detecting the amount of drink present in the vessel, timing means, activated by said detecting means, and means for indicating the progressive deterioration of the organoleptic characteristics which is activated by said timing means.

Also forming an object of the present finding is a procedure for checking the deterioration of the organoleptic characteristics of a drink, characterised in that it consists of periodically detecting the amount of a drink present in a vessel and of subsequently indicating the progressive deterioration of the organoleptic properties of said drink, as a function of both said detected amount and of the time which has passed since the preparation of said drink.

Other characteristics of the present invention are defined, moreover, in the subsequent claims.

Further characteristics and advantages of the invention will become clearer from the description of a preferred but not exclusive embodiment of the device and of the procedure for checking the deterioration of the organoleptic characteristics of a drink according to the finding, which are illustrated to indicate and not for limiting purposes in the attached drawings, wherein:
- figure 1 shows a schematic perspective frontal view of a device according to the finding;
- figure 2 shows a schematic view of an optical indicator which indicates the quality of the drink, in different possible configurations;
- figure 3 shows a diagram of the procedure according to the finding;
- figure 4 shows a graph indicating the deterioration of the organoleptic qualities of the drink as the amount of drink in the vessel varies, and
- figure 5 shows a table which indicates the reference times at which the LEDs are switched off.

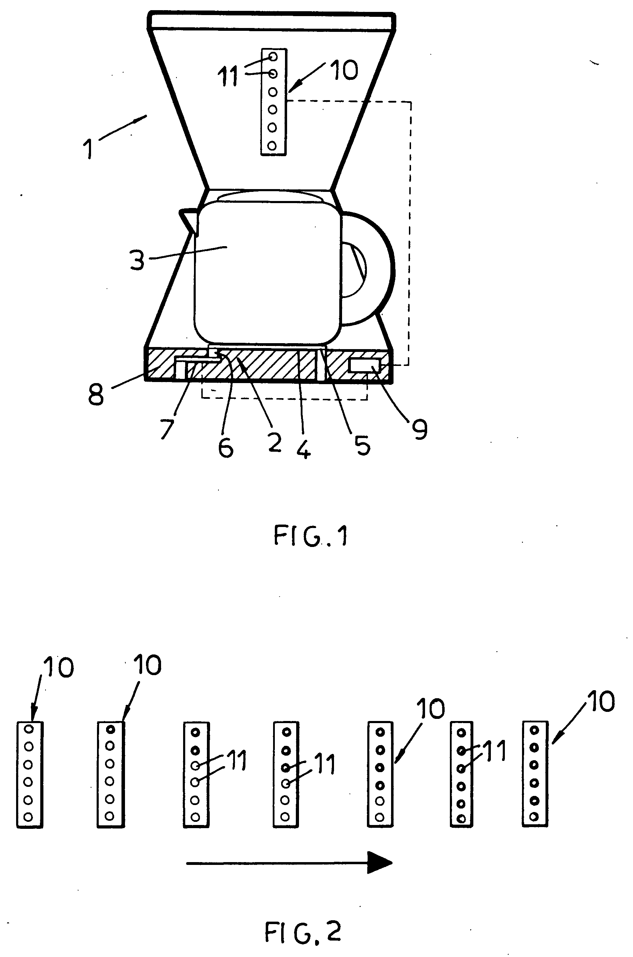

With reference to figures 1 and 2, a device for checking the deterioration of the organoleptic characteristics of a drink is shown, wholly indicated with the reference number 1.

The checking device 1 comprises detecting means 2 of the amount of drink present in the vessel 3.

The detecting means 2 preferably comprises a plate 4 which is hinged at 5 to the device 1 and having an appendage 6 associated with a load cell 7 contained in a base 8 of the device 1.

The load cell 7 is electrically connected to timing means 9.

The timing means 9 comprises an electronic circuit electrically connected both to the load cell 7, and to indicating means 10 of the progressive deterioration of the organoleptic characteristics of the drink.

Such indicating means 10 comprises an optical indicator equipped with a plurality of elements 11, such as LEDs, which can be individually activated by the electronic circuit.

For example the indicator 11 comprises six green LEDs and one LED which can be both green and red.

In a different embodiment the detecting means 2 comprises a probe which measures the temperature in many points of the vessel 3, so as to detect the level of liquid inside of it.

Moreover, the base 8 contains an electrical resistor (not shown) suitable for constantly heating the vessel 3 to keep the liquid contained in it hot.

In a constructive variant the electronic circuit of the timing means is also suitable for interrupting the electrical supply to such a resistor when the quality of the drink becomes inferior, for example when only one LED 11 is left lit (this being a green or, preferably, red LED).

The procedure which is object of the present finding consists of periodically detecting the amount of a drink present in a vessel and of subsequently indicating the progressive deterioration of the organoleptic properties of the same drink, as a function both of such a detected amount and of the time which has passed since the drink was first prepared.

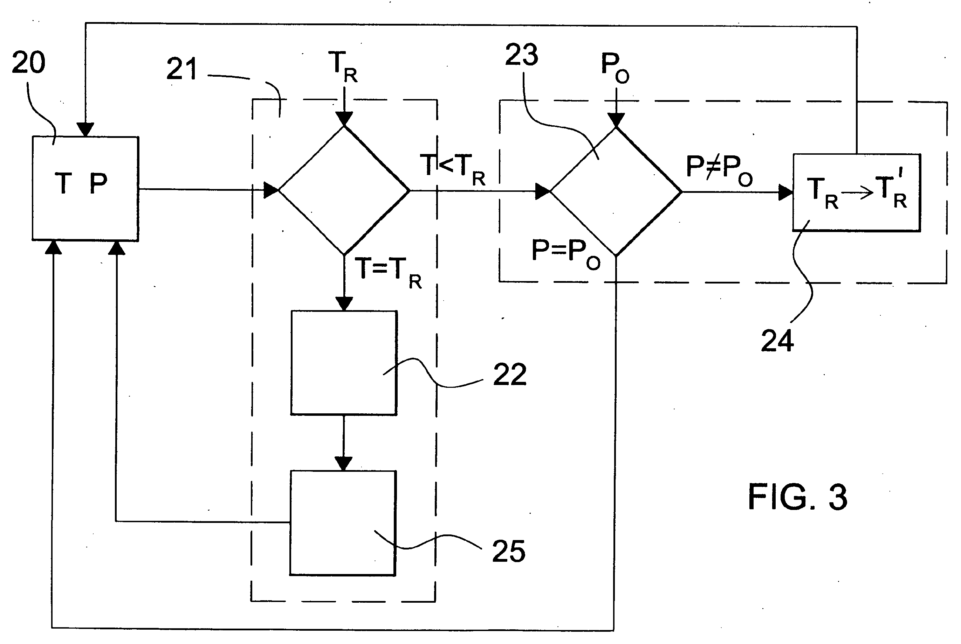

The operation of the device for checking the deterioration of the organoleptic characteristics of a drink is described hereafter with reference to figures 3-4.

When it is switched on the device is reset and, in particular, a signal T proportional to the time passed since the vessel 3 was placed on the plate or since the drink was prepared, and a signal P proportional to the amount of drink contained in the vessel itself 3 are set to zero.

The load cell 7 carries out a measurement every 30 seconds to determine the end of the percolation.

When the load cell detects the same weight (with a margin of ± 20 grams) in three consecutive measurements the percolation is adjudged to be finished and a first operation step 20 begins.

When the load cell 7 detects a zero or negative measurement for three consecutive measurements, however, the device switches off automatically.

In the first operation step 20 the signal T is updated and the signal P is measured.

The updating of the signal T is carried out by increasing it by a predetermined amount; the signal P, on the other hand, is updated by carrying out a new measurement of the weight of the vessel 3.

In this step the load cell 7 measures the weight of the vessel 3 every 60 seconds.

In a second operating step 21 the signal T is elaborated and is compared to a reference value Tr.

When T reaches the reference value Tr (and is, thus, equal to it) the configuration of the indicating means 10 is modified (22); subsequently the operation starts again from the first step 20.

For example, supposing that when the device 1 is activated the LEDs 11 are all lit, the output of the indicating means is modified switching off one of the LEDs 11 (or changing its colour, e.g. green to red).

If, on the other hand, the signal T is less than the reference value Tr a third operation step 23 begins.

In the third operating step 23 the signal P is elaborated so that when it is detected that some of the drink has been removed, the reference value Tr is modified (24) into a different reference value Tr'; subsequently the first step 20 starts all over again.

For example the third operating step 23 is carried out comparing the input value P with a previously memorised value P0.

If P=P0 (i.e. drink has not been removed) the procedure starts again from the first step 20.

If, on the other hand, P<P0 (i.e. drink has been removed) the reference value Tr is modified to the different reference value Tr'.

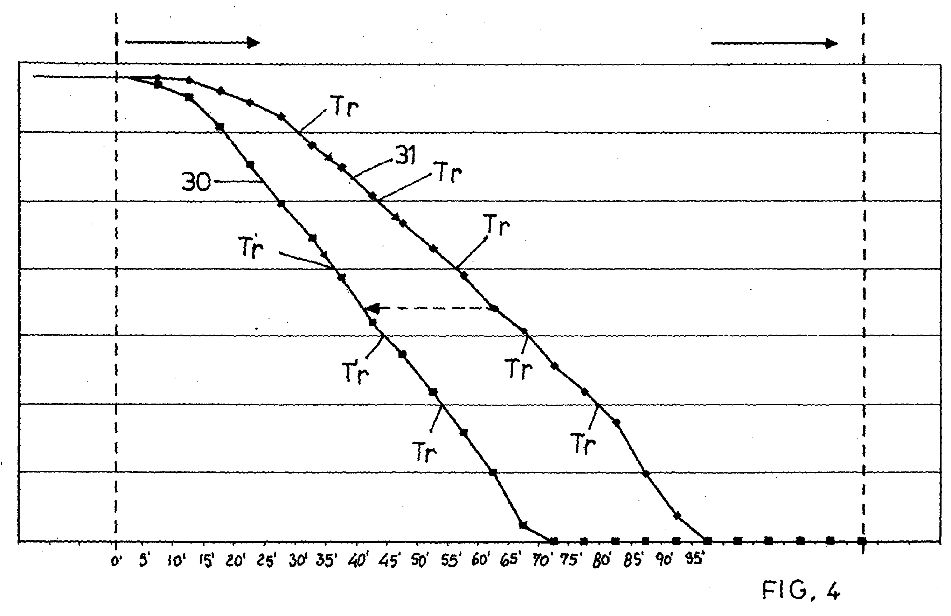

In figure 4 a diagram which shows two curves indicating the progressive deterioration of the organoleptic characteristics of the drink is shown as an example.

A first curve 30 indicates the progressive deterioration of the organoleptic properties of 250 grams of drink, a curve 31, on the other hand, indicates the progressive deterioration of the organoleptic properties of 1250 grams of drink.

If one assumes that one starts with 1250 grams of drink and that one removes 1000 grams, leaving only 250 grams of drink in the vessel 3, the electronic circuit will initially follow the curve 31 (with the reference values Tr indicated therein), however, when the reduction in amount of drink is detected, the electronic circuit will follow the curve 30 (with different reference values Tr' indicated therein).

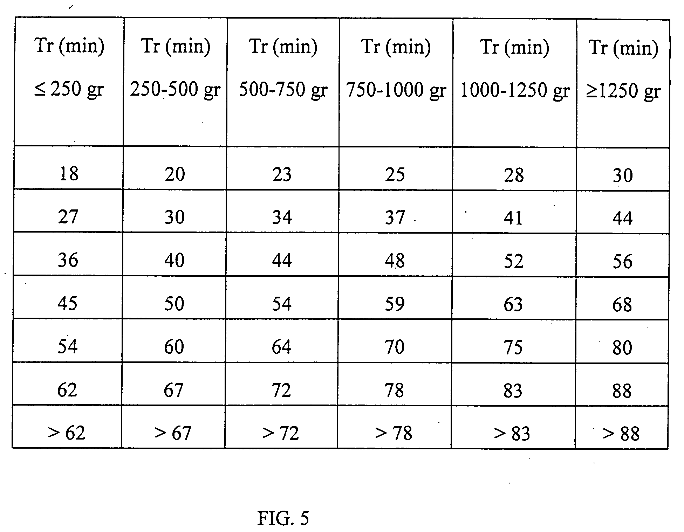

In figure 5 the values of Tr relative to the amount of drink detected are indicated.

Subsequently, the first step 20 starts all over again.

Moreover, in a preferred example, the second operating, step 21 comprises a fourth step 25 wherein, when it is detected that the signal T has reached the reference value Tr, the same reference value Tr is increased with a smaller increment to that of the previous interval.

In practice, as is clear from the table of figure 5, for the Tr values relative to 1250 grams of drink the first interval lasts 30 minutes, the second 14 minutes, the third 12 minutes, etc.

All of the steps are cyclically repeated until the load cell 7 detects a zero or negative weight for three consecutive measurements.

Moreover, in a different embodiment of the device 1 according to the finding, the resistor for heating the drink is associated with two mechanical thermostats set at two different temperatures.

For example, a first mechanical thermostat is set to heat the resistor to 135°C whereas the second mechanical thermostat is set to heat the resistor to 145°C.

The two thermostats are connected to the electronic circuit of the timing means 9, which selects one or other of the two thermostats according to the amount of drink which is detected by the load cell 7.

In practice, therefore, the load cell 7 carries out a measurement of the weight of the drink contained in the vessel 3. When such a measurement is less than a minimum selected value the first timer is activated and the resistor is heated to 135°C. However, when the measurement is higher than the minimum value the second timer is activated and the resistor is heated to 145°C.

Of course, embodiments with more than two thermostats or with a single electronic thermostat which is suitable for working at different temperatures which can be chosen by the electronic circuit according to the measurement taken by the load cell 7 are also possible.

In a particularly advantageous embodiment the device according to the finding can be incorporated in a coffee maker, for example for filter coffee.

In practice it has been noted how the device and the procedure for checking the deterioration of the organoleptic characteristics of a drink are particularly advantageous because they allow the quality of the drink to be checked before tasting it and, if necessary to tip it away.

The device and the procedure for checking the deterioration of the organoleptic characteristics of a drink thus conceived are susceptible to numerous modifications and variants, all falling within the inventive concept; moreover, all the details can be replaced by technically equivalent elements. In practice, the materials used, as well as the sizes, can be whatever according to the requirements and the state of the art.

## Claims

1. Device (1) for checking the deterioration of the organoleptic characteristics of a drink, **characterised in that** it comprises means for detecting (2) the amount of drink present in a vessel (3), timing means (9), activated by said detecting means (2), and indicating means (10) of the progressive deterioration of the organoleptic characteristics, activated by said timing means (9).

2. Device (1) according to claim 1, **characterised in that** said timing means (9) comprises at least one electronic circuit electrically connected to said detecting means (2) and to said indicating means (10).

3. Device (1) according to one or more of the previous claims, **characterised in that** said detecting means (2) comprises at least one load cell (7).

4. Device (1) according to one or more of the previous claims, **characterised in that** the detecting means (2) comprises at least one probe which measures the temperature of said vessel (3) so as to detect the level of the liquid inside of it.

5. Device (1) according to one or more of the previous claims, **characterised in that** said indicating means (10) comprises at least one optical indicator.

6. Device according to one or more of the previous claims, **characterised in that** said optical indicator (10) comprises a plurality of elements (11) which can be individually activated by said electronic circuit.

7. Device according to one or more of the previous claims, **characterised in that** said electronic circuit of said timing means (9) is suitable for interrupting the electrical supply to an electrical resistor suitable for heating said vessel (3) to keep the liquid contained in it hot, when the quality of the drink becomes inferior.

8. Device (1) according to one or more of the previous claims, **characterised in that** said electrical resistor is associated with at least one thermostat suitable for controlling the heating of said electrical resistor alternatively at at least two different temperatures according to the amount of coffee detected in said vessel (3).

9. Procedure for checking the deterioration of the organoleptic characteristics of a drink, **characterised in that** it consists of periodically detecting the amount of a drink present in a vessel and of then indicating, as a function both of said detected amount and of the time which has passed since the preparation of said drink, the progressive deterioration of the organoleptic properties of said drink.

10. Procedure according to claim 9, **characterised in that** it comprises the following steps which are repeated cyclically:
- a first step (20) wherein at least one signal T proportional to the time and a signal P proportional to the amount of drink contained in a vessel (3) are generated or updated;
- a second step (21) wherein said signal T proportional to the time is elaborated so that, if the signal has reached a reference value Tr, the configuration of the indicating means (10) is modified (22);
- a third step (23) wherein said signal P proportional to the amount of drink is elaborated so that, if it is detected that drink has been removed, said reference value Tr is modified (24) to a different reference value Tr'.

11. Procedure according to claim 10, **characterised in that** said second step (21) comprises a fourth step (25) wherein, when it is detected that said signal T has reached said reference value Tr, the same reference value Tr is increased with an increment less than that of the previous interval.

12. Coffee maker **characterised in that** it comprises a device for checking the deterioration of the organoleptic characteristics of a drink according to one or more of the claims from 1 to 8.

13. Device and procedure for checking the deterioration of the organoleptic characteristics of a drink as described and claimed.
